# EUROPÄISCHE PATENTANMELDUNG

(11) **EP 2 910 129 A1**
(43) Veröffentlichungstag der Anmeldung: **26.08.2015**
(21) Anmeldenummer: 14000633.9
(22) Anmeldetag: 21.02.2014
(51) Int. Cl.: A23L 1/015, C11B 3/00, C11B 3/04, C11B 3/16

(54) **Zusammensetzung für die enzymatische Ölentschleimung**

(71) Anmelder: Clariant Produkte (Deutschland) GmbH, 65929 Frankfurt am Main (DE)
(72) Erfinder: Sohling, Ulrich, 85356 Freising (DE); Suck, Kirstin, 80939 München (DE); Bubenheim, Paul, 22359 Hamburg (DE)

(57) **Zusammenfassung**

Die Erfindung betrifft eine Zusammensetzung umfassend mindestens ein Phospholipid-spaltendes Enzym und mindestens eine Protease. Weiterhin betrifft die Erfindung ein Verfahren zur Entschleimung von Triglycerid-haltigen Zusammensetzungen unter Verwendung der erfindungsgemäßen Zusammensetzung sowie die Verwendung der erfindungsgemäßen Zusammensetzung zur Entschleimung von Triglycerid-haltigen Zusammensetzungen.

## Beschreibung

Die Erfindung betrifft eine Zusammensetzung umfassend mindestens ein Phospholipid-spaltendes Enzym und mindestens eine Protease. Weiterhin betrifft die Erfindung ein Verfahren zur Entschleimung von Triglycerid-haltigen Zusammensetzungen unter Verwendung der erfindungsgemäßen Zusammensetzung sowie die Verwendung der erfindungsgemäßen Zusammensetzung zur Entschleimung von Triglycerid-haltigen Zusammensetzungen.

Triglyceride, die aus pflanzlichen Rohstoffen gewonnen werden, insbesondere rohe Pflanzenöle, enthalten Phosphatide, eiweiß-und kohlenhydrathaltige Stoffe, pflanzliche Schleimstoffe sowie kolloidale Verbindungen, die die Haltbarkeit des Öls stark herabsetzen und die Ausbeute des aufgereinigten Öles verringern. Diese Stoffe müssen daher entfernt werden.

Bei der Raffination von Pflanzenölen werden die unerwünschten Begleitstoffe entfernt, die das Öl unbrauchbar machen würden. Man unterscheidet zwischen chemischer und physikalischer Raffination. Die chemische Raffination besteht aus den Prozessen 1. Entschleimung, bei der Phospholipide und Metallionen aus dem Öl entfernt werden, 2. Neutralisation mit Lauge, bei der die Fettsäuren extrahiert werden, 3. Bleichung zur Entfernung von Farbstoffen, weiterer Metallionen und restlichen Schleimstoffen, 4. Desodorierung, eine Wasserdampfdestillation, bei der weitere Verbindungen entfernt werden, die den Geruch- und den Geschmack des Öls beeinträchtigen. Bei der physikalischen Raffination wird die Entsäuerung zusammen mit der Desodorierung am Ende des Raffinationsprozesses durchgeführt.

Die Entschleimung der Öle kann durch Extraktion der Phospholipide mit Wasser oder einer wässrigen Lösung einer Säure erfolgen, die Ca²⁺- und Mg²⁺-Ionen komplexiert, wie z.B. Zitronensäure oder Phosphorsäure. Häufig führt man hierbei zunächst eine wässrige, sogenannte Vorentschleimung durch, mit der die wasserlöslichen Phospholipide entfernt werden. Man spricht hierbei von hydratisierbaren Phospholipiden.

Die Thematik der hydratisierbaren und nicht-hydratisierbaren Phospholipide ist beispielsweise beschrieben in Nielsen, K., Composition of difficultly extractable soy bean phosphatides, J. Am. Oil. Chem. Soc. 1960, 37, 217-219 und A.J. Dijkstra, Enzymatic degumming, Eur. J. Lipid Sci. Technol. 2010, 112, 1178-1189. Dabei handelt es sich insbesondere um Phosphatidyl-Cholin und Phosphatidyl-Inositol. Die Behandlung mit verdünnten wässrigen Calcium- und Magnesium-komplexierenden Säuren, wie z.B. Zitronensäure oder Phosphorsäure, führt nach dem Stand der Technik dazu, dass nicht-hydratisierbare Phospholipide in hydratisierbare Phospholipide überführt werden. Eine Reduktion des Phosphorgehaltes auf 10 oder weniger ppm Phosphor im Öl muss für Nahrungsmittelanwendungen regelmäßig nachgewiesen werden (nach dem Stand der Technik bestimmt durch ICP/AES-Analyse des Öls). Für Öle, die beispielsweise zur Herstellung von Biodiesel eingesetzt werden, werden sogar noch strengere Anforderungen gestellt. Dort ist nach der EU-Norm der Phosphorgehalt des Biodiesels auf 5 ppm begrenzt und es ist zweckmäßig, die Phosphorreduktion bereits ölseitig durchzuführen.

Ein Nachteil der konventionellen Ölentschleimungsprozesse liegt darin, dass sowohl die wässrige.Vorentschleimung als auch die Behandlung mit wässrigen Säuren zu Ölverlusten führen, die dadurch verursacht sind, dass die in das Wasser überführten Phospholipide Emulgatoren darstellen, die einen Teil des Pflanzenöls in der wässrigen Phase emulgieren, wodurch Pflanzenöl verloren geht. Diese Verluste können im Bereich weniger Prozente bezogen auf das ursprünglich eingesetzte Rohöl liegen. Als Faustregel gilt, dass mit je zwei Molekülen Phospholipid etwa ein Triglyceridmolekül emulgiert wird (beschrieben in WO 08/094847).

Die sogenannte enzymatische Entschleimung vermeidet mehrere Nachteile der bestehenden Verfahren bzw. verbessert die Extraktionsverfahren.

Die Verwendung von Phospholipasen, vor allem Phospholipase A, zur Entschleimung von Rohölen ist beispielsweise in der EP 0513709 B1 (sogenannter Enzymax^{®}-Prozess der Firma Lurgi, Frankfurt) beschrieben. Es wird davon ausgegangen, dass die Abspaltung einer Fettsäure zu einem Lysolecithin führt, welches eine wesentlich geringere Emulgierkapazität für Öl hat und auch eine wesentlich höhere Wasserlöslichkeit besitzt. Dadurch wird sowohl die Ölausbeute erhöht als auch die Wasserlöslichkeit der schwer hydratisierbaren Phospholipide verbessert. Der aktuelle Stand der Technik zur enzymatischen Ölentschleimung ist zusammengefasst in den beiden Artikeln von A.J. Dijkstra, Recent developments in edible oil processing, Eur. J. Lipid Sci. Technol. 2009, 111, 857-864, und dem Artikel von A.J. Dijkstra, Enzymatic degumming, Eur. J. Lipid Sci. Technol. 2010, 112, 1178-1189. Dort werden die Vor- und Nachteile der einzelnen Phospholipasen für die enzymatische Ölentschleimung diskutiert und auch die Vorbehandlungsmethoden mit unterschiedlichen Säuren angeführt.

Vom Standpunkt der Ölausbeute aus wäre es am günstigsten für die enzymatische Entschleimung, eine hochwirksame Phospholipase C einzusetzen, die als Produkt ein Diglycerid liefert, welches im Öl löslich ist, und einen Phosphatidylrest, wie z.B. Phosphatidyl-Cholin (ausgehend von Lecithin), welcher sehr gut wasserlöslich ist. Solche Enzyme hat die Firma Verenium in der US 7,226,771 beschrieben. Im Review-Artikel von Dijkstra zum Thema "Enzymatic degumming" wird als Nachteil dieses Systems angeführt, dass es nicht alle Phospholipide umsetzt, sondern lediglich Phosphatidyl-Cholin und Phosphatidyl-Inositol, während die schwer hydratisierbaren Ethanolamine und Phosphatidsäuren unangetastet bleiben. Dieser Nachteil hat dazu geführt, dass in Folgeentwicklungen die Phospholipase C entweder mit Phospholipasen A oder mit Lipidacyltransferasen kombiniert wurde. Eine Kombination von Phospholipasen A mit Phospholipasen C zur Ölentschleimung ist in der WO 08/094847 beschrieben. In dieser Patentschrift wird angeführt, dass die Mischung von Phospholipase A und Phospholipase C einerseits zu einem synergistischen Effekt bei der Ölausbeute führt, andererseits sich damit sehr niedrige Phosphorgehalte im Öl mit verträglichen Reaktionszeiten einstellen lassen.

Die Kombination von Phospholipase C mit Lipidacyltransferasen ist in der WO 2009/081094 beschrieben. Auch hier wird angeführt, dass die Kombination der Acyltransferase mit der Phospholipase C zu einer Erhöhung der Ölausbeute führt. Eine weitere Variante der enzymatischen Ölentschleimung stellt die enzymatische Behandlung der abgetrennten Schleimphase dar, nachdem das Öl nach konventionellen Verfahren wie z.B. mit Wasser und/oder Zitronensäure entschleimt wurde. Durch diese Behandlung ist es möglich, einen Teil des in der Schleimphase emulgierten Pflanzenöles wiederzugewinnen. Dieser Prozess ist beispielsweise auch in dem Review-Artikel A.J. Dijkstra, Enzymatic degumming, Eur. J. Lipid Sci. Technol. 2010, 112, 1178-1189 S. 1184 diskutiert.

Des Weiteren beschreibt die PCT/EP2013/053199 ein Verfahren, bei dem Rohöl mit einer Enzymkombination aus einem Phospholipid-spaltenden Enzym und einer Glykosidase entschleimt wird. Ein weiteres Verfahren des Standes der Technik, das in der EP 13166529.1 beschrieben wird, setzt im Rahmen einer enzymatischen Entschleimung eine Phosphatase ein.

Der Aspekt der Nachhaltigkeit des Einsatzes von Phospholipasen gegenüber anderen Entschleimungsmethoden ist schließlich im Artikel L. De Maria & J. Vind & K. M. Oxenbøll & A. Svendsen & S. Patkar, Phospholipases and their industrial applications, Appl Microbiol Biotechnol (2007) 74:290-300 S. 96 und 97 beschrieben. Am Beispiel einer Ölmühle, die von einem konventionellen Entschleimungsprozess auf einen Prozess mit Phospholipase A umgestellt wurde und in der pro Jahr 266 000 t Sojaöl aufgereinigt werden, wurde gezeigt, dass dort pro Jahr 120000 GJ Energie und 12000 t CO₂ Äquivalente eingespart werden können. Die eingesparten CO₂-Äquivalente entsprechen den Emissionen von 1600 durchschnittlichen Erdbewohnern.

Aufgrund der weltweiten Zunahme des Verbrauchs an Speiseöl und der immer stärker werdenden Nutzung von Pflanzenölen als Rohstoffe für die chemische Industrie und als Treibstoff besteht ständig weiterer Bedarf, die Entschleimung von Triglycerid-haltigen Zusammensetzungen, insbesondere von Pflanzenölen und/oder Pflanzenölschleimen, zu verbessern.

Die Erfinder der vorliegenden Anmeldung haben sich daher die Aufgabe gestellt, ein alternatives enzymatisches Verfahren zu den aus dem Stand der Technik bekannten Verfahren zur Entschleimung von Triglycerid-haltigen Zusammensetzungen, insbesondere rohen Pflanzenölen zu entwickeln, mit denen der Phosphorgehalt des zu entschleimenden Triglycerids bzw. der Triglycerid-haltigen Zusammensetzung weiter verringert, die Ölausbeute erhöht und/oder die Reaktionsgeschwindigkeit der enzymatischen Entschleimung erhöht wird. Gleichzeitig soll dieses Verfahren eine wirtschaftliche Durchführung im großtechnischen Maßstab ermöglichen.

Enzymaktivität wird im Rahmen der vorliegenden Erfindung als eine chemische Reaktion definiert, die von einem oder mehreren katalytischen Proteinen (Enzymen) katalysiert wird. Dabei wird ein Enzymsubstrat zu einem oder mehreren Produkten umgesetzt. Bestimmte Enzyme oder Enzymzusammensetzungen besitzen eine oder sogar mehrere Enzymaktivitäten. Ein reines Enzym kann z. B. mehr als eine Reaktion katalysieren (Umsetzung von einem Substrat zu Produkt(en)), hat daher mehr als eine Enzymaktivität. Viele Enzymzusammensetzungen sind keine biochemisch reinen Produkte und haben somit mehrere Enzymaktivitäten. Die Enzymaktivität steht im Zusammenhang mit der Reaktionsgeschwindigkeit. Sie gibt an, wie viel aktives Enzym sich in einer Enzymzusammensetzung befindet. Die Einheiten der Enzymaktivität sind Unit (U), wobei 1 U definiert ist als diejenige Menge Enzym, welche unter angegebenen Bedingungen ein Mikromol Substrat pro Minute umsetzt: 1 U = 1 µmol/min.

Diese Aufgabe wurde durch eine Zusammensetzung gelöst umfassend eine erste Enzymkomponente, umfassend mindestens ein Phospholipid-spaltendes Enzym, sowie eine zweite Enzymkomponente umfassend mindestens eine Protease ("erfindungsgemäße Zusammensetzung").

Im Rahmen der vorliegenden Erfindung handelt es sich bei dem "Phospholipid-spaltenden Enzym" bevorzugt um eine Phospholipase, die in der Lage ist, entweder einen Fettsäurerest oder einen Phosphatidylrest oder eine Kopfgruppe von einem Phospholipid abzuspalten. Des Weiteren handelt es sich bei dem "Phospholipid-spaltenden Enzym" bevorzugt um eine Acyltransferase, bei der die Abspaltung des Fettsäurerests mit einer Übertragung dieses Restes, gefolgt von einer Esterbildung, mit einem freien Sterol in der Ölphase verbunden ist.

Phospholipasen sind Enzyme, die zur Gruppe der Hydrolasen gehören und die die Esterbindung von Phospholipiden hydrolysieren. Phospholipasen werden nach ihrer Regioselektivität bei Phospholipiden in 5 Gruppen eingeteilt:
Phospholipasen A1 (PLA1), die die Fettsäure in der sn1-Position unter Bildung des 2-Lysophospholipids abspalten.
Phospholipasen A2 (PLA2), die die Fettsäure in der sn2-Position unter Bildung des 1-Lysophospholipids abspalten.
Phospholipasen C (PLC), die einen Phosphorsäuremonoester abspalten.
Phospholipasen D (PLD), die die Kopfgruppe abspalten oder austauschen.
Phospholipasen B (PLB), die die Fettsäure sowohl in der sn1-Position als auch in der sn2-Position unter Bildung eines 1,2-Lysophospholipids abspalten.

Unter einer Acyltransferase versteht man im Rahmen der vorliegenden Erfindung ein Enzym, das Acylgruppen, z. B. Fettsäuren aus einem Phospholipid, auf einen geeigneten Akzeptor, z. B. ein Sterol, unter Bildung eines Esters überträgt.

In einer bevorzugten Ausführungsform betrifft die vorliegende Erfindung eine Zusammensetzung, bei der die erste Enzymkomponente ausgewählt wird aus der Gruppe bestehend aus Phospholipase A1, Phospholipase A2, Phospholipase C, Phospholipase B, Phospholipase D, Acyltransferase und deren Mischungen. Die Enzyme können dabei von einem beliebigen Organismus (z.B. auch isoliert aus einem thermophilen Organismus) oder einer synthetischen Quelle stammen. Die Enzyme können dabei tierischen Ursprungs, z.B. aus dem Pankreas, pflanzlichen Ursprungs oder mikrobiellen Ursprungs sein, z. B. aus Hefe, Pilzen, Algen oder Bakterien stammen. Es ist im Rahmen der vorliegenden Erfindung auch möglich, dass innerhalb der Enzymkomponenten jeweils Enzyme gleicher Art eingesetzt werden, die jedoch aus unterschiedlichen Quellen bzw. Spezies stammen. Ebenso sind rekombinant hergestellte, chimäre Fusionsproteine aus zwei oder mehreren verschiedenen Spezies mit enzymatischer Aktivität umfasst.

Im Rahmen der vorliegenden Erfindung werden Phospholipase A1, Phospholipase A2, Phospholipase C, Phospholipase B, Phospholipase D, Acyltransferase und deren Mischungen bevorzugt aus folgenden Spezies eingesetzt: Schweinepankreas, Rinderpankreas, Schlangengift, Bienengift, Aspergillus, Bacillus, Citrobacter, Clostridium, Dictyostelium, Edwardsiella, Enterobacter, Escherichia, Erwinia, Fusarium, Klebsiella, Listeria, Mucor, Naja, Neurospora, Pichia, Proteus, Pseudomonas, Providencia, Rhizomucor, Rhizopus, Salmonella, Sclerotinia, Serratia, Shigella, Streptomyces, Thermomyces, Trichoderma, Trichophyton, Whetzelinia, Yersinia.

Besonders bevorzugt werden Phospholipase A1, Phospholipase A2, Phospholipase C, Phospholipase B, Phospholipase D, Acyltransferase und deren Mischungen aus *Aspergillus awamori, Aspergillus foetidus, Aspergillus fumigatus, Aspergillus japonicus, Aspergillus niger, Aspergillus oryzae, Bacillus alvei, Bacillus amyloliquefaciens, Bacillus anthracis, Bacillus atrophaeus, Bacillus cereus, Bacillus circulans, Bacillus coagulans, Bacillus larvae, Bacillus laterosporus, Bacillus megaterium, Bacillus natto, Bacillus pasteurii, Bacillus pumilus, Bacillus sphaericus, Bacillus sporothermodurans, Bacillus subtilis, Bacillus thuringiensis, Bacillus pseudoanthracis, Citrobacter amalonaticus, Citrobacter braakii, Citrobacter farmeri, Citrobacter freundii, Citrobacter gillenii, Citrobacter koseri, Citrobacter murliniae, Citrobacter rodentium, Citrobacter sedlakii, Citrobacter werkmanii, Citrobacter youngae, Clostridium perfringens, Dictyostelium discoideum, Dictyostelium mucoroides, Dictyostelium polycephalum, Edwardsiella hoshinae, Edwardsiella ictaluri, Edwardsiella tarda, Enterobacter amnigenus, Enterobacter aerogenes, Enterobacter cloacae, Enterobacter gergoviae, Enterobacter intermedius, Enterobacter pyrinus, Escherichia albertii, Escherichia blattae, Escherichia coli, Escherichia fergusonii, Escherichia hermannii, Escherichia senegalensis, Escherichia vulneris, Erwinia amylovora, Erwinia aphidicola, Erwinia billingiae, Erwinia carotovora, Erwinia herbicola, Erwinia oleae, Erwinia mallotivora, Erwinia papayae, Erwinia persicina, Erwinia piriflorinigrans, Erwinia psidii, Erwinia pyrifoliae, Erwinia rhapontici, Erwinia tasmaniensis, Erwinia toletana, Erwinia tracheiphila, Fusarium avenaceum, Fusarium avenaceum, Fusarium chlamydosporum, Fusarium coeruleum, Fusarium culmorum, Fusarium dimerum, Fusarium incarnatum, Fusarium heterosporum, Fusarium moniliforme, Fusarium napiforme, Fusarium oxysporum, Fusarium poae, Fusarium sporotrichiella, Fusarium tricinctum, Fusarium proliferatum, Fusarium sacchari, Fusarium solani, Fusarium sporotrichioides, Fusarium subglutinans, Fusarium tabacinum, Fusarium verticillioides, Klebsiella oxytoca, Klebsiella mobilis, Klebsiella singaporensis, Klebsiella granulomatis, Klebsiella pneumoniae, Klebsiella variicola, Listeria monocytogenes, Mucor amphibiorum, Mucor circinelloides, Mucor hiemalis, Mucor indicus, Mucor javanicus, Mucor mucedo, Mucor paronychius, Mucor piriformis, Mucor subtilissimus, Mucor racemosus, Naja mossambica, Neurospora Africana, Neurospora crassa, Neurospora discrete, Neurospora dodgei, Neurospora galapagosensis, Neurospora intermedia, Neurospora lineolata, Neurospora pannonica, Neurospora sitophila, Neurospora sublineolata, Neurospora terricola, Neurospora tetrasperma, Pichia barkeri, Pichia cactophila, Pichia cecembensis, Pichia cephalocereana, Pichia deserticola, Pichia eremophilia, Pichia exigua, Pichia fermentans, Pichia heedii, Pichia kluyveri, Pichia kudriavzevii, Pichia manshurica, Pichia membranifaciens, Pichia nakasei, Pichia norvegensis, Pichia orientalis, Pichia pastoris (Komagataella pastoris), Pichia pseudocactophila, Pichia scutulata, Pichia sporocuriosa, Pichia terricola, Proteus hauseri, Proteus mirabilis, Proteus myxofaciens, Proteus penneri, Proteus vulgaris, Pseudomonas aeruginosa, Pseudomonas fluorescens, Pseudomonas putida, Pseudomonas syringae, Providencia rettgeri, Providencia stuartii, Rhizomucor endophyticus, Rhizomucor miehei, Rhizomucor pakistanicus, Rhizomucor pusillus, Rhizomucor tauricus, Rhizomucor variabilis, Rhizopus arrhizus, Rhizopus azygosporus, Rhizopus circinans, Rhizopus japonicus, Rhizopus microsporus, Rhizopus nigricans, Rhizopus oligosporus, Rhizopus oryzae, Rhizopus schipperae, Rhizopus sexualis, Rhizopus stolonifer, Rhizopus artocarpi, Salmonella bongori, Salmonella enterica, Salmonella typhimurium, Sclerotinia borealis, Sclerotinia homoeocarpa, Sclerotinia libertiana, Sclerotinia minor, Sclerotinia ricini, Sclerotinia sclerotiorum, Sclerotinia spermophila, Sclerotinia trifoliorum, Serratia entomophila, Serratia ficaria, Serratia fonticola, Serratia grimesii, Serratia liquefaciens, Serratia marcescens, Serratia odorifera, Serratia plymuthica, Serratia proteamaculans, Serratia quinivorans, Serratia rubidaea, Serratia symbiotica, Shigella dysenteriae, Shigella flexneri, Shigella boydii, Shigella sonnei, Streptomyces achromogenes, Streptomyces ambofaciens, Streptomyces aureofaciens, Streptomyces avermitilis, Streptomyces carcinostaticus, Streptomyces cervinus, Streptomyces clavuligerus, Streptomyces coelicolor, Streptomyces coeruleorubidus, Streptomyces davawensis, Streptomyces fradiae, Streptomyces griseus, Streptomyces hygroscopicus, Streptomyces lavendulae, Streptomyces lincolnensis, Streptomyces natalensis, Streptomyces nodosus, Streptomyces noursei, Streptomyces peuceticus, Streptomyces platensis, Streptomyces rimosus, Streptomyces spectabilis, Streptomyces toxytricini, Streptomyces venezuelae, Streptomyces violaceoniger, Streptomyces violaceoruber, Thermomyces lanuginosa, Trichoderma harzianum, Trichoderma koningii, Trichoderma longibrachiatum, Trichoderma pseudokoningii, Trichoderma reesei, Trichoderma viride, Trichophyton concentricum, Trichophyton eboreum, Trichophyton equinum, Trichophyton gourvilii, Trichophyton kanei, Trichophyton megninii, Trichophyton mentagrophytes, Trichophyton phaseöliforme, Trichophyton raubitschekii, Trichophyton rubrum, Trichophyton schoenleinii, Trichophyton simii, Trichophyton soudanense, Trichophyton terrestre, Trichophyton tonsurans, Trichophyton vanbreuseghemii, Trichophyton verrucosum, Trichophyton violaceum, Trichophyton yaoundei, Whetzelinia sclerotiorum, Yersinia aldovae, Yersinia aleksiciae, Yersinia bercovieri, Yersinia enterocolitica, Yersinia frederiksenii, Yersinia intermedia, Yersinia kristensenii, Yersinia massiliensis, Yersinia mollaretii, Yersinia pestis, Yersinia pseudotuberculosis, Yersinia rohdei, Yersinia ruckeri, Yersinia similis eingesetzt.*

In einer besonders bevorzugen Ausführungsform werden Phospholipase A₁, Phospholipase A₂, Phospholipase B, Phospholipase C und/oder Phospholipase D eingesetzt, die aus *Aspergillus niger, Aspergillus oryzae, Bacillus cereus, Bacillus megaterium, Bacillus subtilis, Citrobacter freundii, Enterobacter aerogenes, Enterobacter cloacae, Edwardsiella tarda, Erwinia herbicola, Escherichia coli Clostridium perfringens, Dictyostelium discoideum, Fusarium oxysporium, Klebsiella pneumoniae, Listeria monocytogenes, Mucor javanicus, Mucor mucedo, Mucor subtilissimus, Naja mossambica, Neurospora crassa, Pichia pastoris (Komagataella pastoris), Pseudomonas spezies, Proteus vulgaris, Providencia stuartii, Rhizomucor pusillus, Rhizopus arrhizus, Rhizopus japonicus, Rhizopus stolonifer Salmonella typhimurium, Serratia marcescens, Serratia liquefaciens, Sclerotinia libertiana, Shigella flexneri, Streptomyces violaceoruber, Trichophyton rubrum, Thermomyces lanuginosus, Trichoderma reesei, Whetzelinia sclerotiorum, Yersinia enterocolitica,* Schweinepankreas, Rinderpankreas, Schlangengift oder Bienengift stammen.

Das mindestens eine Enzym der ersten Enzymkomponente kann dabei aus der gleichen oder aus unterschiedlichen Quellen stammen, bevorzugt aus einem oder aber auch aus mehreren der vorgenannten Organismen, besonders bevorzugt aus *Aspergillus niger, Aspergillus oryzae, Fusarium oxysporium, Naja mossambica, Pichia pastoris (Komagataella pastoris), Streptomyces violaceoruber, Thermomyces lanuginosus, Trichoderma reesei,* Schweinepankreas oder Rinderpankreas.

Unter dem Begriff "Protease" werden im Rahmen der vorliegenden Erfindung ein oder mehrere Enzyme oder Enzymzusammensetzungen aus der Enzymklasse 3.4 (Peptidhydrolasen) verstanden. Dies schließt die Begriffe Peptidasen und oder Proteinasen mit ein. Proteasen katalysieren die Hydrolyse von Peptidbindungen. Die Enzyme können dabei tierischen Ursprungs, z.B. aus Magenschleimhaut, pflanzlichen Ursprungs oder mikrobiellen Ursprungs, z. B. aus Hefe, Pilzen, Algen oder Bakterien stammen. Insbesondere kann es sich bevorzugt um ein oder mehrere Enzyme der folgenden Protease-Enzymklassen handeln: Aminopeptidasen, Aspartatendopeptidasen, Dipeptidasen, Dipeptidylpeptidasen, Tripeptidylpeptidasen, Peptidyldipeptidasen, Carboxypeptidase des Typs Serin, Metallocarboxypeptidasen, Carboxypeptidasen des Typs Cystein, Omegapeptidasen, Serin-Endopeptidasen, Cystein-Endopeptidasen, Asparagin-Endopeptidasen, Metalloendopeptidasen, Threonin-Endopeptidasen, Endopeptidasen, wobei besonders bevorzugt Aspartatendopeptidasen, Serin-Endopeptidasen oder Metalloendopeptidasen eingesetzt werden.

Bevorzugt werden im Rahmen der vorliegenden Erfindung die genannten Proteasen aus folgenden Spezies eingesetzt: Magenschleimhaut aus dem Säugetier, Schweinepankreas, Rinderpankreas, Aspergillus, Bacillus, Citrobacter, Clostridium, Dictyostelium, Edwardsiella, Enterobacter, Escherichia, Erwinia, Fusarium, Klebsiella, Listeria, Mucor, Naja, Neurospora, Pichia, Proteus, Pseudomonas, Providencia, Rhizomucor, Rhizopus, Salmonella, Sclerotinia, Serratia, Shigella, Streptomyces, Thermomyces, Trichoderma, Trichophyton, Whetzelinia, Yersinia.

Besonders bevorzugt werden die Protease und deren Mischungen aus *Aspergillus awamori, Aspergillus foetidus, Aspergillus fumigatus, Aspergillus japonicus, Aspergillus niger, Aspergillus oryzae, Aspergillus sojae, Bacillus alvei, Bacillus amyloliquefaciens, Bacillus anthracis, Bacillus atrophaeus, Bacillus cereus, Bacillus circulans, Bacillus coagulans, Bacillus larvae, Bacillus laterosporus, Bacillus megaterium, Bacillus natto, Bacillus pasteurii, Bacillus pumilus, Bacillus sphaericus, Bacillus sporothermodurans, Bacillus subtilis, Bacillus thuringiensis, Bacillus pseudoanthracis, Bacillus polymyxa, Citrobacter amalonaticus, Citrobacter braakii, Citrobacter farmeri, Citrobacter freundii, Citrobacter gillenii, Citrobacter koseri, Citrobacter murliniae, Citrobacter rodentium, Citrobacter sedlakii, Citrobacter werkmanii, Citrobacter youngae, Clostridium perfrin gens, Dictyostelium discoideum, Dictyostelium mucoroides, Dictyostelium polycephalum, Edwardsiella hoshinae, Edwardsiella ictaluri, Edwardsiella tarda, Enterobacter amnigenus, Enterobacter aerogenes, Enterobacter cloacae, Enterobacter gergoviae, Enterobacter intermedius, Enterobacter pyrinus, Escherichia albertii, Escherichia blattae, Escherichia coli, Escherichia fergusonii, Escherichia hermannii, Escherichia senegalensis, Escherichia vulneris, Erwinia amylovora, Erwinia aphidicola, Erwinia billingiae, Erwinia carotovora, Erwinia herbicola, Erwinia oleae, Erwinia mallotivora, Erwinia papayae, Erwinia persicina, Erwinia piriflorinigrans, Erwinia psidii, Erwinia pyrifoliae, Erwinia rhapontici, Erwinia tasmaniensis, Erwinia toletana, Erwinia tracheiphila, Fusarium avenaceum, Fusarium avenaceum, Fusarium chlamydosporum, Fusarium coeruleum, Fusarium culmorum, Fusarium dimerum, Fusarium incarnatum, Fusarium heterosporum, Fusarium moniliforme, Fusarium napiforme, Fusarium oxysporum, Fusarium poae, Fusarium sporotrichiella, Fusarium tricinctum, Fusarium proliferatum, Fusarium sacchari, Fusarium solani, Fusarium sporotrichioides, Fusarium subglutinans, Fusarium tabacinum, Fusarium verticillioides, Klebsiella oxytoca, Klebsiella mobilis, Klebsiella singaporensis, Klebsiella granulomatis, Klebsiella pneumoniae, Klebsiella variicola, Listeria monocytogenes, Mucor amphibiorum, Mucor circinelloides, Mucor hiemalis, Mucor indicus, Mucor javanicus, Mucor mucedo, Mucor paronychius, Mucor piriformis, Mucor subtilissimus, Mucor racemosus, Naja mossambica, Neurospora Africana, Neurospora crassa, Neurospora discrete, Neurospora dodgei, Neurospora galapagosensis, Neurospora intermedia, Neurospora lineolata, Neurospora pannonica, Neurospora sitophila, Neurospora sublineolata, Neurospora terricola, Neurospora tetrasperma, Pichia barkeri, Pichia cactophila, Pichia cecembensis, Pichia cephalocereana, Pichia deserticola, Pichia eremophilia, Pichia exigua, Pichia fermentans, Pichia heedii, Pichia kluyveri, Pichia kudriavzevii, Pichia manshurica, Pichia membranifaciens, Pichia nakasei, Pichia norvegensis, Pichia orientalis, Pichia pastoris, Pichia pseudocactophila, Pichia scutulata, Pichia sporocuriosa, Pichia terricola, Proteus hauseri, Proteus mirabilis, Proteus myxofaciens, Proteus penneri, Proteus vulgaris, Pseudomonas aeruginosa, Pseudomonas fluorescens, Pseudomonas putida, Pseudomonas syringae, Providencia rettgeri, Providencia stuartii, Rhizomucor endophyticus, Rhizomucor miehei, Rhizomucor pakistanicus, Rhizomucor pusillus, Rhizomucor taüricus, Rhizomucor variabilis, Rhizopus arrhizus, Rhizopus azygosporus, Rhizopus circinans, Rhizopus japonicus, Rhizopus microsporus, Rhizopus nigricans, Rhizopus oligosporus, Rhizopus oryzae, Rhizopus schipperae, Rhizopus sexualis, Rhizopus stolonifer, Rhizopus artocarpi, Salmonella bongori, Salmonella enterica, Salmonella typhimurium, Sclerotinia borealis, Sclerotinia homoeocarpa, Sclerotinia libertiana, Sclerotinia minor, Sclerotinia ricini, Sclerotinia sclerotiorum, Sclerotinia spermophila, Sclerotinia trifoliorum, Serratia entomophila, Serratia ficaria, Serratia fonticola, Serratia grimesii, Serratia liquefaciens, Serratia marcescens, Serratia odorifera, Serratia plymuthica, Serratia proteamaculans, Serratia quinivorans, Serratia rubidaea, Serratia symbiotica, Shigella dysenteriae, Shigella flexneri, Shigella boydii, Shigella sonnei, Streptomyces achromogenes, Streptomyces ambofaciens, Streptomyces aureofaciens, Streptomyces avermitilis, Streptomyces carcinostaticus, Streptomyces cervinus, Streptomyces clavuligerus, Streptomyces coelicolor, Streptomyces coeruleorubidus, Streptomyces davawensis, Streptomyces fradiae, Streptomyces griseus, Streptomyces hygroscopicus, Streptomyces lavendulae, Streptomyces lincolnensis, Streptomyces natalensis, Streptomyces nodosus, Streptomyces noursei, Streptomyces peuceticus, Streptomyces platensis, Streptomyces rimosus, Streptomyces spectabilis, Streptomyces toxytricini, Streptomyces venezuelae, Streptomyces violaceoniger, Streptomyces violaceoruber, Thermomyces lanuginosa, Trichoderma harzianum, Trichoderma koningii, Trichoderma longibrachiatum, Trichoderma pseudokoningii, Trichoderma reesei, Trichoderma viride, Trichophyton concentricum, Trichophyton eboreum, Trichophyton equinum, Trichophyton gourvilii, Trichophyton kanei, Trichophyton megninii, Trichophyton mentagrophytes, Trichophyton phaseoliforme, Trichophyton raubitschekii, Trichophyton rubrum, Trichophyton schoenleinii, Trichophyton simii, Trichophyton soudanense, Trichophyton terrestre, Trichophyton tonsurans, Trichophyton vanbreuseghemii, Trichophyton verrucosum, Trichophyton violaceum, Trichophyton yaoundei, Whetzelinia sclerotiorum, Yersinia aldovae, Yersinia aleksiciae, Yersinia bercovieri, Yersinia enterocolitica, Yersinia frederiksenii, Yersinia intermedia, Yersinia kristensenii, Yersinia massiliensis, Yersinia mollaretii, Yersinia pestis, Yersinia pseudotuberculosis, Yersinia rohdei, Yersinia ruckeri, Yersinia similis* eingesetzt.

Bevorzugt werden im Rahmen der vorliegenden Erfindung Proteasen aus folgenden Spezies eingesetzt: Proteasen aus Magenschleimhaut eines Säugetiers, Schweinepankreas, Rinderpankreas, *Aspergillus niger, Aspergillus oryzae, Aspergillus saitoi, Aspergillus sojae, Bacillus cereus, Bacillus licheniformis, Bacillus amyloliquifaciens, Bacillus polymyxa, Bacillus subtilis, Escherichia coli Clostridium perfringens, Pichia pastoris (Komagataella pastoris), Pseudomonas spezies, Rhizomucor pusillus, Rhizopus arrhizus, Rhizopus japonicus, Rhizopus stolonifer, Salmonella typhimurium, Serratia marcescens, Serratia liquefaciens, Streptomyces griseus, Streptomyces violaceoruber, Thermomyces lanuginosus, Trichoderma reesei, Yersinia enterocolitica.*

Die mindestens eine Protease der zweiten Enzymkomponente kann dabei aus der gleichen oder aus unterschiedlichen Quellen stammen, bevorzugt aus einem oder aber auch aus mehreren der vorgenannten Organismen.

In einer bevorzugten Ausführungsform wird die Menge des/der Enzyms/der Enzyme der ersten Enzymkomponente im Bereich von 1 ppm bis 1000 ppm, bevorzugter von 1 bis 250 ppm, besonders bevorzugt im Bereich von 5 bis 200 ppm in Bezug auf die Ölmenge gewählt. In einer weiteren bevorzugten Ausführungsform wird die Enzymaktivität der zweiten Enzymkomponente im Bereich von 1 ppm bis 1000 ppm, bevorzugter von 1 bis 250 ppm, besonders bevorzugt im Bereich von 5 bis 200 ppm in Bezug auf die Ölmenge gewählt.

In einer bevorzugten Ausführungsform wird die Enzymaktivität des/der Enzyms/der Enzyme der ersten Enzymkomponente im Bereich von 0,01 bis 10 units/g Öl gewählt, bevorzugter im Bereich von 0,1 bis 5 units/g Öl, besonders bevorzugt im Bereich von 0,2 bis 3 units/g Öl und am meisten bevorzugt im Bereich von 0,3 bis 2 units/g Öl. In einer weiteren bevorzugten Ausführungsform wird die Enzymaktivität der zweiten Enzymkomponente im Bereich von 0,01 bis 10 units/g Öl, bevorzugt 0,1 bis 5 units/g Öl, und besonders bevorzugt im Bereich von 0,2 bis 3 units/g Öl, und am meisten bevorzugt im Bereich von 0,3 bis 2 units/g Öl gewählt. (Unit: Internationale Einheit für Enzymaktivität; 1 Unit entspricht dem Substratumsatz von 1 µmol/min).

Besonders bevorzugt sind im Rahmen der vorliegenden Erfindung Zusammensetzungen, bei denen das Verhältnis der Enzymaktivität der ersten Enzymkomponente zu der Enzymaktivität der zweiten Enzymkomponente im Bereich von 0,001 : 20 bis 20 : 0,001 liegt, bevorzugt im Bereich von 0,1 : 10 bis 10 : 0,1, weiter bevorzugt im Bereich von 0,25 : 7,5 bis 7,5 : 0,25, weiter bevorzugt von 0,5 : 5 bis 5 : 0,5 und am meisten bevorzugt im Bereich von 1 : 1 bis 1 : 5.

Mit der Einhaltung des erfindungsgemäß bevorzugten Verhältnisses der ersten Enzymkomponente zur zweiten Enzymkomponente lässt sich das Volumen der Schleimphase weiter reduzieren. Dies bedeutet eine Steigerung der Ölausbeute.

Die Enzyme der ersten und/oder zweiten Enzymkomponente können beispielsweise gefriergetrocknet und in entsprechendem Enzympuffer (Standardpuffer für jedes Enzym sind in der Literatur beschrieben) gelöst verwendet werden, z.B. Citratpuffer 0,1 M, pH 5 oder Acetatpuffer 0,1 M, pH 5. In einer bevorzugten Ausführungsform werden die Enzyme in Enzympuffer aufgenommen und dem Rohöl zugesetzt. Um eine bessere Löslichkeit der Enzyme - insbesondere in den Phospholipase-enthaltenden erfindungsgemäßen Zusammensetzungen - zu erreichen, ist auch der Zusatz von organischen Lösungsmitteln möglich. Diese finden Anwendung z.B. in der Auftrennung der Phospholipide und sind in der Literatur beschrieben. Bevorzugt verwendet werden unpolare organische Lösungsmittel wie z.B. Hexan oder Aceton oder Mischungen, bevorzugt in einer Menge von 1 bis 30 Gew.-% (Beispiele möglicher Lösungsmittel sind beschrieben in der EP 1531182 A2).

In einer weiteren bevorzugten Ausführungsform wird die erste und/oder zweite Enzymkomponente in geträgerter Form eingesetzt. Im Rahmen der vorliegenden Erfindung bevorzugte Trägermaterialien sind anorganische Trägermaterialien, wie z.B. Kieselgele, Fällungskieselsäuren, Silikate oder Alumosilikate, und organische Trägermaterialien, wie z.B. Methacrylate oder Ionentauscherharze. Die Trägermaterialien erleichtern die Wiederverwertbarkeit der Enzyme aus der Öl-Wasser-Emulsion in einem folgenden Verfahrensschritt und tragen zur Wirtschaftlichkeit des Verfahrens bei.

In einer ebenfalls bevorzugten Ausführungsform umfasst die erfindungsgemäße Zusammensetzung ein oder mehrere weitere Bestandteile, besonders bevorzugt ausgewählt aus der Gruppe bestehend aus Citratpuffer und Acetatpuffer.

Die Erfinder der vorliegenden Zusammensetzung haben überraschend herausgefunden, dass eine Kombination der vorstehend definierten Enzymkomponenten (erste und zweite Enzymkomponente) besonders wirkungsvoll und effektiv die Emulgierbarkeit von Triglyceriden in wässrigen Phasen verringert. Daher kann die erfindungsgemäße Zusammensetzung besonders vorteilhaft für die Entschleimung von Triglycerid-haltigen Zusammensetzungen wie rohem Pflanzenöl oder auch von Pflanzenölschleim eingesetzt werden.

Die vorliegende Erfindung betrifft daher in einem weiteren Aspekt ein Verfahren zur Entschleimung von Triglycerid-haltigen Zusammensetzungen umfassend die Schritte
a) in-Kontakt-Bringen der Triglycerid-haltigen Zusammensetzung mit der erfindungsgemäßen Zusammensetzung wie vorstehend definiert;
b) Abtrennen der Schleimstoffe von der Triglycerid-haltigen Zusammensetzung.

Erstaunlicherweise wurde hierbei gefunden, dass es durch das erfindungsgemäße Verfahren gelingt, den Phospholipidgehalt der Triglycerid-haltigen Zusammensetzung gegenüber dem reinen Einsatz von Phospholipid-spaltendem Enzym weiter abzusenken, die Ölausbeute zu erhöhen, die Reaktionsgeschwindigkeit bei der enzymatischen Entschleimung zu erhöhen, das Schleimvolumen zu erniedrigen und/oder die Abtrennbarkeit der gebildeten Schleimphase zu verbessern. Dabei wird der Phosphorwert auf unter 20 ppm gesenkt, besonders bevorzugt auf unter 10 ppm, ganz besonders bevorzugt auf unter 4 ppm Phosphor.

Weiterhin ist es mit dem erfindungsgemäßen Verfahren möglich, den Calcium- und Magnesiumgehalt der Triglycerid-haltigen Zusammensetzung, insbesondere rohen Pflanzenöls, auf unter 20 ppm zu senken, besonders bevorzugt auf unter 15 ppm, ganz besonders bevorzugt auf unter 10 ppm, ebenfalls bevorzugt auf unter 8 ppm und am meisten bevorzugt auf unter 4 ppm. In einer weiteren bevorzugten Ausführungsform wird der Calcium- und Magnesiumgehalt auf unter 3 ppm gesenkt.

Das Verfahren der vorliegenden Erfindung ist dabei besonders vorteilhaft, da durch den Einsatz der Protease die Wirkung des Phospholipid-spaltenden Enzyms verbessert wird. Durch den Einsatz der Protease erfolgt eine Erniedrigung der Viskosität der Ölschleimphase sowie eine Erhöhung der Beweglichkeit der Phospholipide. Auch wird die Zugänglichkeit der Phospholipid-Moleküle für das Phospholipid-spaltende Enzym erhöht, die sich an der Grenzfläche Schleimphase/Öl befinden.

Durch das erfindungsgemäße Kombinieren von mindestens einem Phospholipid-spaltenden Enzym mit mindestens einer Protease ist es zudem möglich, die Dosierung der Phospholipid-spaltenden Enzyme, wie z.B. der Phospholipase A1 oder A2 gegebenenfalls kombiniert mit Phospholipase C, zu verringern und so neben den oben angeführten Vorteilen für den Prozess auch Kosten zu sparen.

Unter dem Begriff "Triglyceride" werden im Rahmen der vorliegenden Erfindung dreifache Ester des Glycerins mit Fettsäuren verstanden, die den Hauptbestandteil von natürlichen Fetten und Ölen darstellen, sei es pflanzlichen oder tierischen Ursprungs. Triglycerid-haltige Zusammensetzungen im Sinne der vorliegenden Erfindung umfassen pflanzliche oder tierische Fette und Öle, und deren Mischungen sowohl untereinander als auch mit synthetischen bzw. modifizierten Fetten und Ölen. Die Begriffe werden nachfolgend näher definiert.

Unter dem Begriff "Pflanzenöl" wird im Rahmen der vorliegenden Erfindung jedes Öl pflanzlichen Ursprungs verstanden. Bevorzugte besonders geeignete Öle sind Sojaöl, Rapsöl, Canolaöl, Sonnenblumenöl, Olivenöl, Palmöl, Jatrophaöl, Leindotteröl, Baumwollsaatöl und deren Mischungen. Besonders geeignet sind "rohe Pflanzenöle". Die Bezeichnung "roh" bezieht sich dabei darauf, dass das Öl noch keinem Entschleimungs-, Neutralisations-, Bleichungs- und/oder Desodorierungsschritt unterzogen wurde. Es ist im Rahmen des erfindungsgemäßen Verfahrens auch möglich, dass eine Mischung mehrerer Rohöle eingesetzt wird oder vorbehandelte, z.B. vorentschleimte und/oder vorkonditionierte Öle mit den Enzymen behandelt werden.

Unter "Schleimphase", "Schleimstoffe", "Pflanzenölschleim" versteht man im Rahmen der vorliegenden Erfindung alle Stoffe, die nach Behandlung mit einer säurehaltigen und/oder wässrigen Lösung aus der Triglycerid-haltigen Zusammensetzung als schwere Phase ausfallen (Michael Bokisch: Fats and Oils Handbook, AOCS Press, Champaign, Illinois, 1998, Seite 428-444. Die Begriffe "Schleimphase", "Schleimstoffe", "Pflanzenölschleim" werden dabei im Rahmen der vorliegenden Erfindung synonym verwendet. Die Verwendung dieses Pflanzenölschleims als Ausgangsmaterial ist insbesondere für die Gewinnung von Lecithin von Bedeutung, da Lecithin ein wesentlicher Bestandteil des Pflanzenölschleims ist.

Unter dem Begriff "Vorentschleimung" bzw. "Nassentschleimung" wird im Rahmen der vorliegenden Erfindung eine Behandlung des Rohöls mit Wasser oder einer wässrigen Säurelösung verstanden, um wasserlösliche Phospholipide weitestgehend aus dem Öl zu entfernen. Die Begriffe "Vorentschleimung" und "Nassentschleimung" werden dabei im Rahmen der vorliegenden Erfindung synonym verwendet. Auch kann im Rahmen einer Vor- bzw. Nassentschleimung nach der Säurezugabe ggf. eine Zugabe von Alkali erfolgen, um die Säure zu neutralisieren. Vor der Enzymzugabe erfolgt die Abtrennung der wässrigen Phase. Nach einer Vorentschleimung wird der Phosphorgehalt im Rohöl von ca. 500-1500 ppm, z.B. für Soja und Raps auf unter 200 ppm im vorentschleimten Öl gesenkt. Durch die Vorentschleimung kann z.B. Lecithin aus der entstandenen Schleimphase gewonnen werden bzw. die Schleimphase als Futtermittel aufgearbeitet werden. Der Nachteil der Abtrennung der wässrigen Phase bzw. der Senkung des Phosphorgehaltes ist jedoch ein Ausbeuteverlust bezüglich des Öls. Die in die wässrige Phase übergehenden Phosphatide wirken emulgierend und führen dazu, dass ein Teil des Öls in der wässrigen Phase emulgiert und mit dieser abgetrennt wird. Anschließend kann das Öl enzymatisch weiterbehandelt werden.

Unter dem Begriff "Vorkonditionierung" des Öls wird im Rahmen der vorliegenden Erfindung die Zugabe von Wasser bzw. einer wässrigen Säurelösung zu dem unbehandelten Öl verstanden. Anschließend wird durch Zugabe von Alkali, z. B. Natronlauge, ein pH-Wert eingestellt, bei dem die folgende enzymatische Reaktion stattfindet. Idealerweise wird der für die Enzymreaktion optimale pH-Wert von 3,5 bis 7 eingestellt. Anschließend erfolgt jedoch keine Abtrennung der wässrigen Phase, sondern unmittelbar die Zugabe der Enzyme. Die vorhandenen Schleimstoffe verbleiben also vorerst im Öl bzw. in der Emulsion. Die Abtrennung der wässrigen Phase und damit der Enzyme erfolgt erst nach Einwirkung der Enzyme auf das (ggfs. vorkonditionierte) Rohöl.

In einer bevorzugten Ausführungsform kann eine Zugabe von Wasser bzw. einer wässrigen Säurelösung und ggf. von Alkali zur Neutralisierung der Säure zum Rohöl im Sinne einer Vorkonditionierung erfolgen, jedoch unterbleibt die Abtrennung der wässrigen Phase vor der Zugabe der Enzyme. Durch den Verzicht auf den Abtrennungsschritt vor der Zugabe der Enzyme ist eine weitere Steigerung der Ölausbeute möglich. Eine Steigerung der Ölausbeute um einen Prozentpunkt hat eine enorme wirtschaftliche Bedeutung, da dieses Prozent ca. 400.000 t Öl entspricht, bezogen auf die jährliche Produktion von z. B. Sojaöl. Das erfindungsgemäße Verfahren erlaubt somit in dieser bevorzugten Ausführungsform die unmittelbare Verwendung von Rohölen aus Soja bzw. Raps mit Phosphorgehalten von 100 bis 1.500 ppm Phosphor. Überdies stellt es eine Vereinfachung des Verfahrens dar, weil der Trennschritt vor Enzymzugabe entfällt.

In einer weiteren bevorzugten Ausführungsform werden in Schritt a) des erfindungsgemäßen Verfahrens - abgesehen von etwaigen bereits vorhandenen Emulgatoren, wie z.B. Lecithin - keine zusätzlichen Emulgatoren, wie z.B. Natriumdocecylsulfat (SDS), zugegeben. Ebenso kommt das erfindungsgemäße Verfahren bevorzugt ohne die Zugabe von Salzen, wie z.B. Calciumchlorid (CaCl₂) aus.

Für die Entschleimung von Sojaöl oder Rapsöl oder Sonnenblumenöl ist insbesondere eine Kombination der Phospholipase A1 aus *Thermomyces lanuginosus* oder *Fusarium oxysporium* und/oder der Phospholipase A2 aus Schweinepankreas oder Rinderpankreas oder *Trichoderma reesei* oder *Streptomyces violaceoruber* oder *Aspergillus niger* und/oder Phospholipase C aus *Pichia pastoris* mit einer Protease aus Magen oder *Bacillus amyloliquifaciens* oder *Bacillus subtilis* oder *Bacillus licheniformis* oder *Aspergillus niger* oder *Aspergillus oryzae* bevorzugt.

Das "in-Kontakt-Bringen" gemäß Schritt a) des erfindungsgemäßen Verfahrens kann im Rahmen des erfindungsgemäßen Verfahrens auf jede Weise erfolgen, die dem Fachmann als für den erfindungsgemäßen Zweck geeignet bekannt ist. Bevorzugte Art des in-Kontakt-Bringens gemäß Schritt a) des erfindungsgemäßen Verfahrens ist dabei ein Vermischen der Triglycerid-haltigen Zusammensetzung und der erfindungsgemäßen Zusammensetzung.

Nach dem in-Kontakt-Bringen der Triglycerid-haltigen Zusammensetzung mit der erfindungsgemäßen Zusammensetzung gemäß Schritt a) des erfindungsgemäßen Verfahrens wird die Mischung aus der Triglycerid-haltigen Zusammensetzung und der erfindungsgemäßen Zusammensetzung bevorzugt gerührt, besonders bevorzugt mit einem Flügelrührer bei 200 bis 800 U/min, bevorzugt 250 bis 600 U/min und am meisten bevorzugt bei 300 bis 500 U/min.

Die Temperatur der Mischung liegt während des in-Kontakt-Bringens gemäß Schritt a) des erfindungsgemäßen Verfahrens bevorzugt im Bereich von 15 bis 99 °C, bevorzugter im Bereich von 20 bis 95 °C, weiter bevorzugt von 22 bis 75 °C, ebenfalls bevorzugt von 25 bis 65 °C, weiter bevorzugt von 30 bis 60 °C und am meisten bevorzugt von 32 bis 55 °C. Dabei muss die Temperatur der Mischung immer so gewählt werden, dass die Denaturierungstemperatur der Enzyme nicht überschritten wird, bevorzugt liegt die Temperatur der Mischung mindestens 5 °C unter der Denaturierungstemperatur der Enzyme bzw.

Die Dauer des in-Köntakt-Bringens gemäß Schritt a) des erfindungsgemäßen Verfahrens liegt dabei bevorzugt im Bereich von 1 Minute bis 12 Stunden, bevorzugter von 5 Minuten bis 10 Stunden, ebenfalls bevorzugt von 10 Minuten bis 6 Stunden, weiter bevorzugt von 10 Minuten bis 3 Stunden.

Der pH Wert der Mischung liegt während des in-Kontakt-Bringens gemäß Schritt a) des erfindungsgemäßen Verfahrens bevorzugt im Bereich von pH 3 bis pH 7,5, bevorzugter im Bereich von pH 4 bis pH 6 und besonders bevorzugt im Bereich von pH 4,0 bis pH 5, 5.

Das in-Kontakt-Bringen gemäß Schritt a) des erfindungsgemäßen Verfahrens der Triglycerid-haltigen Zusammensetzung mit der ersten und der zweiten Enzymkomponente der erfindungsgemäßen Zusammensetzung kann dabei gleichzeitig, oder auch nacheinander erfolgen. Sofern ein in-Kontakt-Bringen nacheinander erfolgt, ist es im Rahmen der vorliegenden Erfindung bevorzugt, wenn die Triglycerid-haltige Zusammensetzung zuerst mit der zweiten Enzymkomponente in Kontakt gebracht wird. In dem Fall, dass die Triglycerid-haltige Zusammensetzung zuerst mit der zweiten Enzymkomponente und dann mit der ersten Enzymkomponente in Kontakt gebracht wird, ist es besonders bevorzugt, wenn nach Zugabe der einen Komponente die Mischung für 30 bis 300 Minuten, bevorzugt 60 bis 240 Minuten, ebenfalls bevorzugt von 70 bis 120 Minuten gerührt wird, bevor eine Zugabe der anderen Komponente erfolgt.

Das "Abtrennen" der Schleimstoffe gemäß Schritt b) des erfindungsgemäßen Verfahrens kann auf jede Weise erfolgen, die dem Fachmann als für den erfindungsgemäßen Zweck geeignet bekannt ist. Bevorzugt erfolgt die Abtrennung jedoch über jegliche Separatoren, wie z. B. Zentrifugen oder Filtrationseinheiten. Bevorzugte Separatoren für das erfindungsgemäße Verfahren sind Düsenseparatoren, Preßschneckenseparatoren, Kammerseparatoren, Tellerseparatoren, Vollmanteltellerseparatoren, Zweiphasen-Dekanter, Dreiphasen-Dekanter, Dreisäulenzentrifugen, Einpufferzentrifugen, Gleitschwingzentrifugen, Schwingzentrifugen, Vollmantel-Schälzentrifugen, Vollmantel-Schneckenzentrifugen, Röhrenzentrifugen, Siebtrommel-Schälzentrifugen, Schubzentrifugen, Siebschneckenzentrifugen, Spänezentrifugen, Stülpfilterzentrifugen und Universalzentrifugen. Bei der Zentrifugation erfolgt eine Phasentrennung der der Triglycerid-haltigen Zusammensetzung, sodass beispielsweise in der bevorzugten Ausführungsform, in der rohes Pflanzenöl als Triglycerid-haltige Zusammensetzung eingesetzt wird, das behandelte Pflanzenöl, die Schleimstoffe und die Enzymzusammensetzung in separaten Phasen vorliegen, die leicht voneinander getrennt werden können.

In einer bevorzugten Ausführungsform wird dabei die Phase enthaltend die Schleimstoffe sowie die Phase enthaltend die erfindungsgemäße Zusammensetzung von dem behandelten Öl abgetrennt. Besonders bevorzugt ist es dabei, wenn gleichzeitig mit den Schleimstoffen die erste und/oder zweite Enzymkomponente abgetrennt wird.

Die Enzyme können nach der Abtrennung regeneriert bzw. gereinigt und beispielsweise in einem neuen Entschleimungsverfahren eingesetzt werden. Die Enzyme können ggfs. über ein Adsorptionsmittel oder über eine entsprechende Säulenchromatographische Methode regeneriert werden. Eine weitere Möglichkeit ist, von der abgetrennten schweren Phase einen Teil in einer weiteren Ölentschleimung des erfindungsgemäßen Verfahrens einzusetzen.

Eine weitere bevorzugte Ausführungsform der vorliegenden Erfindung betrifft zudem ein Verfahren wie vorstehend beschrieben, weiter umfassend den Schritt
c) erneutes in-Kontakt-Bringen der Triglycerid-haltigen Zusammensetzung gemäß Schritt b) mit der ersten und/oder zweiten Enzymkomponente.

Das "in-Kontakt-Bringen" gemäß Schritt c) des erfindungsgemäßen Verfahrens erfolgt dabei bevorzugt unter den gleichen Bedingungen wie vorstehend für Schritt a) des erfindungsgemäßen Verfahrens beschrieben. Dabei wird in einer besonders bevorzugten Ausführungsform die erste und/oder zweite Enzymkomponente vor dem erneuten in-Kontakt-Bringen einer Regeneration unterzogen.

In einer besonders bevorzugten Ausführungsform wird die Triglycerid-haltige Zusammensetzung vor dem in-Kontakt-Bringen gemäß Schritt c) einer Vorkonditionierung wie vorstehend definiert unterzogen.

In einer bevorzugten Ausführungsform des erfindungsgemäßen Verfahrens erfolgt das in-Kontakt-Bringen gemäß Schritt c) wie vorstehend bereits in Bezug auf das in-Kontakt-Bringen gemäß Schritt a) des erfindungsgemäßen Verfahrens definiert.

Die vorliegende Erfindung betrifft in einem weiteren Aspekt die Verwendung der erfindungsgemäßen Zusammensetzung wie vorstehend näher definiert zur Entschleimung von Triglycerid-haltigen Zusammensetzungen.

Nachfolgend sind besonders bevorzugten Ausführungsformen der vorliegenden Erfindung beschrieben, die jedoch den Rahmen der vorliegenden Erfindung in keiner Weise beschränken sondern lediglich der weiteren Veranschaulichung dienen:

### Bevorzugte Ausführungsform A)

a) in-Kontakt-Bringen der Triglycerid-haltigen Zusammensetzung ausgewählt aus rohem Sojaöl, Rapsöl und/oder Sonnenblumenöl mit einer Zusammensetzung umfassend eine erste Enzymkomponente umfassend mindestens einem Enzym ausgewählt aus der Gruppe bestehend aus Phospholipase A1, Phospholipase A2 und Phospholipase C und eine zweite Enzymkomponente umfassend mindestens einem Enzym ausgewählt aus der Gruppe der Endo-Proteasen;
b) Abtrennen der Schleimstoffe von der Triglycerid-haltigen Zusammensetzung durch Zentrifugation.

In einer besonders vorteilhaften Ausführungsform des erfindungsgemäßen Verfahrens gemäß der bevorzugten Ausführungsform A) wird vor Schritt a) des Verfahrens eine sogenannte Vorkonditionierung durchgeführt, indem das rohe Öl in einem eigenen Verfahrensschritt mit einer Menge von 1,5 bis 3 ml/L Öl an organischer Säure, bevorzugt Zitronensäure, vermischt wird. Die Temperatur der Mischung wird hierbei bevorzugt auf 35 bis 60 °C eingestellt, besonders bevorzugt auf 48 °C. Nach einer Reaktionszeit von 30 Minuten bis 2 Stunden, bevorzugt 1 Stunde, wird die Mischung durch Zugabe einer stöchiometrischen Menge Lauge, bevorzugt Natronlauge, in einer Menge von bevorzugt 0,5 bis 2 Mol/l, besonders bevorzugt 1 Mol/l, auf einen pH von 5 eingestellt. Erst danach wird gemäß Schritt a) des erfindungsgemäßen Verfahrens weiter verfahren.

### Bevorzugte Ausführungsform B)

a) in-Kontakt-Bringen der Triglycerid-haltigen Zusammensetzung ausgewählt aus rohem Sojaöl und/oder Rapsöl mit einer Zusammensetzung umfassend eine erste Enzymkomponente umfassend mindestens ein Enzym ausgewählt aus der Gruppe bestehend aus Phospholipase A1, Phospholipase A2 und Phospholipase C und eine zweite Enzymkomponente mit mindestens einem Enzym ausgewählt aus der Gruppe der Endo-Proteasen, wobei es besonders bevorzugt ist, wenn das mindestens eine Enzym der ersten Enzymkomponente auf einem Träger immobilisiert vorliegt;
b) Abtrennen der Schleimstoffe von der Triglycerid-haltigen Zusammensetzung durch Zentrifugation.

In einer besonders vorteilhaften Ausführungsform des erfindungsgemäßen Verfahrens gemäß der bevorzugten Ausführungsform B) ist es besonders bevorzugt, dass die Enzyme der ersten und/oder zweiten Enzymkomponente in einer wässrigen Phase (Puffer bevorzugt im Bereich pH 4,0 bis 5,5, besonders bevorzugt pH 4,0-5,0) in einer Konzentration von 0,05 bis 5 % w/v eingesetzt werden. Das in-Kontakt-Bringen gemäß Schritt a) erfolgt dabei bevorzugt bei einer Temperatur von 22 bis 70 °C, bevorzugter 25 bis 65 °C.

Zudem wird die bei der bevorzugten Ausführungsform B) des erfindungsgemäßen Verfahrens eine Nachentschleimung durch die Zugabe von einer organischen Säure und/oder Lauge (nach Schritt b)) durchgeführt. Die Temperatur der Mischung wird hierbei bevorzugt auf 35 bis 60 °C eingestellt, besonders bevorzugt 48 °C. Nach einer Reaktionszeit von 30 Minuten bis 2 Stunden, bevorzugt 1 Stunde, wird die Mischung durch Zugabe einer Lauge, bevorzugt Natronlauge, in einer Konzentration von bevorzugt 0,5 bis 2 Mol/l, besonders bevorzugt 1 Mol/l, auf einen pH von 5 eingestellt.

### Bevorzugte Ausführungsform C)

a) in-Kontakt-Bringen der Triglycerid-haltigen Zusammensetzung ausgewählt aus Pflanzenölschleim mit einer Zusammensetzung umfassend eine erste Enzymkomponente umfassend mindestens ein Enzym ausgewählt aus der Gruppe bestehend aus Phospholipase A1, Phospholipase A2 und Phospholipase C und eine zweite Enzymkomponente mit mindestens einem Enzym ausgewählt aus der Gruppe der Proteasen, bevorzugt Endo-Proteasen, wobei es besonders bevorzugt ist, wenn das mindestens eine Enzym der ersten Enzymkomponente auf einem Träger immobilisiert vorliegt;
b) Abtrennen der Schleimstoffe von der Triglycerid-haltigen Zusammensetzung durch Zentrifugation.

### Bevorzugte Ausführungsform D)

a) in-Kontakt-Bringen der Triglycerid-haltigen Zusammensetzung ausgewählt aus rohem Sojaöl und/oder rohem Rapsöl mit einer Zusammensetzung umfassend eine erste Enzymkomponente umfassend mindestens ein Enzym ausgewählt aus der Gruppe bestehend aus Phospholipase A1, Phospholipase A2 und Phospholipase C und eine zweite Enzymkomponente umfassend mindestens ein Enzym ausgewählt aus der Gruppe der Proteasen, bevorzugt Endo-Proteasen, wobei es besonders bevorzugt ist, wenn das mindestens eine Enzym der ersten Enzymkomponente auf einem Träger immobilisiert vorliegt;
b) Abtrennen der Schleimstoffe von der Triglycerid-haltigen Zusammensetzung durch Zentrifugation.

In einer besonders vorteilhaften Ausführungsform des erfindungsgemäßen Verfahrens gemäß der bevorzugten Ausführungsform D) ist es besonders bevorzugt, dass vor Schritt a) des Verfahrens eine Vorkonditionierung durchgeführt, indem das rohe Öl in einem eigenen Verfahrensschritt mit einer Menge von 50-1500 ppm an organischer Säure, bevorzugt 100-1200 ppm Zitronensäure, vermischt wird. Die Temperatur der Mischung wird hierbei bevorzugt auf 40 bis 90 °C eingestellt, besonders bevorzugt 45-85 °C. Nach einer Reaktionszeit von 5 Minuten bis 2 Stunden, bevorzugt 10-30 Minuten, wird die Mischung durch Zugabe einer Lauge, bevorzugt Natronlauge, in einer Menge von bevorzugt 0,5 bis 5 Mol/l, besonders bevorzugt 1 Mol/l, konditioniert. Erst danach wird gemäß Schritt a) des erfindungsgemäßen Verfahrens weiter verfahren.

Zudem lassen sich etwaige, noch im der Triglycerid-haltigen Zusammensetzung gelöste und durch die Phospholipasen nicht gespaltene Phosphatidsäuren weiter reduzieren, indem der Caund/oder Mg-Gehalt des gemäß dem Verfahren der vorliegenden Erfindung behandelten Öls reduziert wird. Daher können die oben angeführten, bevorzugten Ausführungsformen A) bis D) des erfindungsgemäßen Verfahrens vorteilhaft noch durch einen Folgeschritt ergänzt werden, bei dem durch nochmalige Zugabe von Komplexierungsmitteln wie z.B. Zitronensäure oder Phosphorsäure oder Oxalsäure oder Milchsäure oder Äpfelsäure der Gehalt an zweiwertigen Ionen und parallel dazu der Gehalt an P im Öl weiter reduziert wird.

### Methoden

Es wurden folgende Analysenmethoden verwendet:
Bestimmung des Phosphorgehaltes in den Pflanzenölen

Die Bestimmung von Phosphor erfolgte durch ICP gemäß DEV E-22.

### Bestimmung des Calcium- und Magnesiumgehaltes in den Pflanzenölen

Die Bestimmung von Calcium und Magnesium erfolgte durch ICP gemäß DEV E-22.

### Bestimmung des Gehalts freier Fettsäuren (FFA)

Bestimmt wird der Gehalt freier Fettsäuren über den Verbrauch an Natriumhydroxid oder Kaliumhydroxid über eine Verseifungsreaktion. Erhalten wird der prozentuale Gehalt freier Fettsäuren im untersuchten Öl. Die Bestimmung erfolgte gemäß DIN 53402 (Methode DGF C-V 2).

### Bestimmung des Schleimvolumens

Mithilfe dieser Bestimmung wird die im Öl enthaltene Schleimphase von enzymatisch unbehandeltem und enzymatisch behandeltem Schleim gemessen. Ein 10 mL Schleuderglas wird auf die Arbeitstemperatur des Reaktionsansatzes erwärmt, die Proben (2X 2 mL) werden eingefüllt und temperiert mindestens 4 Minuten bei 3000 rpm zentrifugiert um die Schleimphase vom Öl zu trennen. Von der oberen Ölphasen werden Proben für die Analytik entnommen. Zu Dokumentationszwecken wird das Ergebnis der Phasenbildung zusätzlich fotografiert.

### Variante 1:

Die zu behandelnde Menge Rohöl, 400 bis 600 g, wird in einen Duran Reaktor DN120 1000 mL eingefüllt und Proben für die Analytik werden abgenommen. Das Öl im Duranreaktor wird mit Hilfe einer Heizplatte auf eine Temperatur von 40 bis 85 °C, insbesondere 48 bis 80 °C, aufgeheizt. Nachdem die Temperatur erreicht ist, wird mit der Vorkonditionierung begonnen. Dafür wird eine definierte, von der Ölmenge abhängige Menge Zitronensäure, (z.B. 1000 ppm), zum Öl zu dosiert. Anschließend wird die Mischung mit einem Ultraturrax^{®} für 1 Minute durchmischt. Als Alternative wird unter Rühren bei etwa 600 rpm 15 Minuten inkubiert, um die Reaktion der Säure abzuwarten. Anschließend wird eine definierte Menge Natronlauge (1 Mol/L, Restmenge zu 2 % v/v, bzw. 3 % v/v abzüglich Wasser aus Säurezugabe und Enzymzugabe) zugegeben, bis ein pH-Wert von ca. 4 erreicht ist und es wird weitere 10 Minuten unter Rühren inkubiert. Nach Abkühlung auf 48°C erfolgt die Zugabe des Enzyms, der Enzym-Mischung oder des Immobilisats, vorzugsweise gelöst in Wasser oder Puffer. Das Enzym wird untergerührt, wofür die Rührerdrehzahl kurzzeitig erhöht werden kann (1 Minute auf 900 rpm), anschließend wird bei niedrigerer Drehzahl weitergerührt.

Die Probenahme erfolgt in definierten Zeitabständen. Die Probe wird mit Hilfe einer Pipette abgenommen, in ein temperiertes Schleuderglas eingefüllt (Temperatur des Reaktionsansatzes) und temperiert mindestens 4 Minuten bei 3000 rpm zentrifugiert um die Schleimphase vom Öl zu trennen. Zu Dokumentationszwecken wird das Ergebnis der Phasenbildung fotografiert, vom Überstand werden Proben zur Bestimmung des Phosphor-, Calcium- und Magnesiumgehaltes abgenommen.

### Variante 2:

In einer weiteren Durchführung werden Phospholipasen und zusätzliche Enzyme in einer geeigneten Kombination als freie Enzyme oder immobilisierte Enzyme zusammen mit einer wässrigen Phase (Enzym-Puffer, pH 4-5) 0,05 bis 5 % w/v, dem Rohöl zugesetzt. Die Emulsion, bestehend aus Wasser, Enzym, eventuell Enzymträgern und Öl, wird durchmischt. Idealerweise wird die Reaktion temperiert zwischen 20 bis 70 °C, besser zwischen 40 bis 65 °C durchgeführt. Anschließend wird die Phasentrennung abgewartet, die Feststoffe setzen sich ab oder können nach einem dem Fachmann bekannten Standardverfahren z.B. über Zentrifugation oder Filtration entfernt werden. Als Nachbehandlung kann das Öl mit verdünnter Säure (z.B. Zitronensäure) oder Lauge nach einem dem Fachmann als "Degumming" bekannten Verfahren restentschleimt werden.

### Variante 3:

In einer weiteren Durchführung wird die Schleimphase mit Enzymen behandelt. Die Schleimphase, welche nach einem dem Fachmann als "degumming" bekannten Verfahren erhalten wird, werden neben Phospholipasen, weitere Enzyme zugesetzt. Diese können sich gelöst in einer wässrigen Phase oder suspendiert in einem organischen Lösungsmittel befinden. Der Ansatz wird idealerweise auf eine Temperatur zwischen 20 bis 70 °C temperiert, besser auf eine Temperatur zwischen 35 bis 60 °C. Der Ansatz wird durchmischt bis der Prozess abgeschlossen ist. Dies kann durch Viskositätsmessungen überprüft werden oder visuell, durch Auflösen der ansonsten festen Schleimphase. Durch Zentrifugation lässt sich eine Phasenseparation erreichen, die einzelnen Phasen können abgetrennt werden. In der Regel besteht die obere Phase aus dem gewonnenen Öl, die mittlere Phase aus den Phospholipiden und die untere Phase ist eine wässrige Phase und enthält die Enzyme. Durch Wiederverwendung der wässrigen Phase lassen sich die Enzyme recyceln und wiederverwenden. Je nach Gehalt zweiwertiger Ionen kann das Öl oder die das Enzym enthaltende Wasserphase durch Zusatz von Komplexierungsmitteln vor der weiteren Verwendung von den Ionen bereinigt werden.

### Beispiele und Figuren:

Die Erfindung wird im Weiteren an Hand von Figuren und Beispielen näher erläutert. Es wird hierbei betont, dass die Beispiele und Figuren lediglich veranschaulichenden Charakter besitzen und besonders bevorzugte Ausführungsformen der vorliegenden Erfindung veranschaulichen. Weder Beispiele noch Figuren beschränken den Rahmen der vorliegenden Erfindung.

### Es zeigen:

- Fig. 1: rohes Rapsöl: Vorkonditionierung mit 3 % Gesamtwasseranteil
- Fig. 2: rohes Rapsöl: Vorkonditionierung mit Zugabe von Enzym PLA1 0,3 Unit/g Öl und 3 % Gesamtwasseranteil
- Fig. 3: rohes Rapsöl: Vorkonditionierung mit Zugabe von Enzym PLA1 0,3 Unit/g Öl und dem Enzym Pepsin 1 Unit/g Öl, 3 % Gesamtwasseranteil

### Beispiel 1:

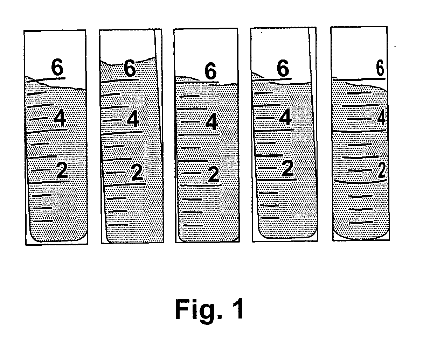

Gemäß Reaktionsvariante 1 wurde ein rohes Rapsöl mit folgenden Ausgangsgehalten verwendet: Phosphor 1200 ppm, Calcium 365 ppm, Magnesium 155 ppm und einem Gehalt an freien Fettsäuren von 1,99 %. Das Rohöl wurde einer Vorkonditionierung mithilfe von wässriger Zitronensäure (1000 ppm) und wässriger Natronlauge (1 Mol/L) unterzogen. Es wurden regelmäßig Proben genommen (siehe Figur 1, Tabelle 1). Am Ende der Reaktion wurde die Schleimphase abzentrifugiert und der Restölgehalt dieser nach Soxhlet bestimmt.

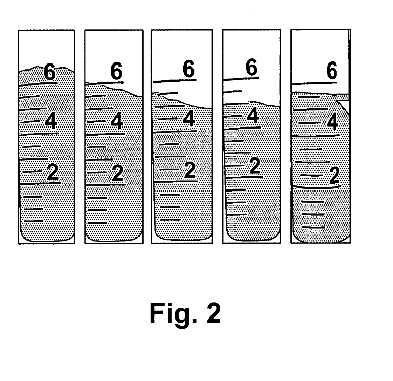

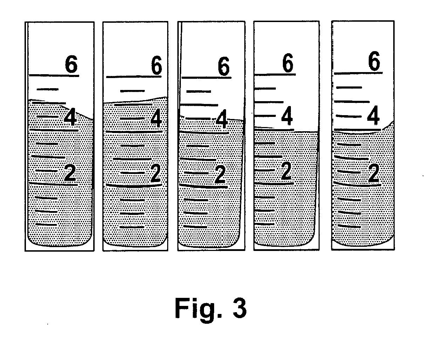

Als Vergleich wurde eben diese Vorkonditionierung mit Zugabe eines Enzyms, Phospholipase A1 aus dem Organismus *Thermomyces lanuginosus* (Sigma-Aldrich), durchgeführt (siehe Figur 2, Tabelle 2). In Figur 3, Tabelle 3 sind Ergebnisse der Vorkonditionierung mit Zusatz des Enzyms PLA1 aus *Thermomyces lanuginosus* und eines weiteren Enzyms, Pepsin aus Porcine *Gastric* mucosa(Sigma-Aldrich) dargestellt.

**Tab. 1 Vorkonditionierung mit 3 % Gesamtwasseranteil, Phosphor-, Calcium-, Magnesium- und FFA Gehalt**

| **Zeit [min]** | **10** | **60** | **120** | **180** | **240** |
|---|---|---|---|---|---|
| **Ca [ppm]** | 76 | 11 | 9,5 | 9,4 | 9,5 |
| **Mg [ppm]** | 31 | 2,8 | 1,7 | 1,6 | 1,7 |
| **P [ppm]** | 247 | 20 | 14 | 13 | 14 |
| **FFA [%]** | 1,73 | | | 1, 68 | 1,72 |
| **Schleim [%]3 min** | 5,8 | 6,5 | 6,0 | 6,0 | 6,0 |

**Tab. 2 Vorkonditionierung mit Zugabe von PLA1 aus Thermomyces lanuginosus 0,3 Units/g Öl und 3 % Gesamtwasseranteil, Phosphor-, Calcium-, Magnesium- und FFA Gehalt**

| **Zeit [min]** | **10** | **60** | **120** | **180** | **240** |
|---|---|---|---|---|---|
| **Ca [ppm]** | 26 | 9,7 | 8,7 | 7,9 | 7,9 |
| **Mg [ppm]** | 9,7 | 2,1 | 1,8 | 1,4 | 1,5 |
| **P [ppm]** | 82 | 17 | 15 | 12 | 12 |
| **FFA [%]** | 1,76 | | | 2,35 | 2,14 |
| **Schleim [%]3 min** | 6,5 | 5,6 | 5,0 | 4,5 | 5,5 |

**Tab. 3 Vorkonditionierung mit Zugabe von PLA1 (aus Thermomyces lanuginosus) 0,3 Units/g Öl und Pepsin 1 Unit/g Öl, 3 % Gesamtwasseranteil, Phosphor, Calcium-, Magnesium- und FFA Gehalt**

| **Zeit [min]** | **10** | **60** | **120** | **180** | **240** |
|---|---|---|---|---|---|
| **Ca [ppm]** | 55 | 10 | 9,3 | 6,4 | 5,7 |
| **Mg [ppm]** | 23 | 3,1 | 3 | 1,9 | 1,6 |
| **P [ppm]** | 199 | 26 | 25 | 16 | 14 |
| **FFA [%]** | 1,86 | | | 2,14 | 2,17 |
| **Schleim [%]3 min** | 4,3 | 5,0 | 4,2 | 4,0 | 4,0 |

Wie aus Figur 1 ersichtlich ist, führt die Anwendung von Säure und Lauge auf das Rohöl als Vorkonditionierung zu einem nicht unerheblichen Schleimvolumen, welches in der Folge trotz Einsatz eines Rührers bei 600 rpm nicht wesentlich abnimmt. Das einzelne Foto entspricht einer Probeentnahme. Die Probeentnahmen erfolgen zu den Zeitpunkten 10, 60, 120, 180 und 240 Minuten(von links nach rechts). In Tabelle 1 sind die zugehörigen analytischen Daten aufgeführt: der Phosphorgehalt sank nach 240 Minuten von 247 ppm auf 14 ppm; die Konzentration der zweiwertigen Ionen Calcium und Magnesium nimmt im Fall des Calciums von 76 ppm auf 9,5 ppm ab; die Konzentration des Magnesiums sinkt von 31 ppm auf 1,7 ppm im Verlauf der Reaktion. Der Gehalt freier Fettsäuren bleibt nahezu unverändert. Die Vorkonditionierung dient als Vorbereitungsreaktion zur Ölentschleimung und gleichzeitig als Referenzbehandlung.

In Figur 2 ist bei Verwendung des Enzyms Phospholipase A1 aus *Thermomyces lanuginosus* (Sigma-Aldrich) eine Abnahme des Schleimvolumens im Verlauf der Reaktion erkennbar (je Messung/ Probenahme ein Foto). Die zugehörigen Daten und die Zeitpunkte der Probenahme sind in Tabelle 2 dargestellt. Aus Tab. 2 ergibt sich eine Abnahme der Calciumkonzentration von 26 ppm auf 7,9 ppm, eine Abnahme der Magnesiumkonzentration von 9,7 ppm auf 1,5 ppm und eine Abnahme des Phosphorgehalts von 82 ppm auf 12 ppm; der Gehalt freier Fettsäuren steigt von 1,76 % auf 2,14 % an, jeweils nach 240 min Reaktionszeit. Aus dem Anstieg im Gehalt der freien Fettsäuren und der Abnahme des Phosphorgehalts lässt sich schließen, dass die PLA1 enzymatisch aktiv ist und folglich die Ölentschleimung erfolgreich funktioniert. Die Zunahme der freien Fettsäure ist ein Zeichen für die Aktivität der PLA1, welche die Fettsäuren aus den Phospholipidmolekülen abspaltet und auch das Schleimvolumen nimmt kontinuierlich ab.

In Figur 3 ist das Volumen der Schleimphase eines mit PLA1 und zusätzlich mit Pepsin behandelten vorkonditionierten Rohöls dargestellt. Aus den dazugehörigen analytischen Daten aus Tabelle 3 ist ersichtlich, dass erstaunlicherweise bereits nach 120 Minuten ein reduziertes Schleimvolumen von 4,2 % erreicht wird, im Vergleich zum Schleimvolumen von 5,0 % bei Einsatz der PLA1 alleine (Tabelle 2). Zusätzlich sind die Ionenwerte (Tabelle 3) vergleichbar mit denen der Reaktion mit der PLA1 alleine, siehe Tabelle 2. Der Gehalt freier Fettsäuren steigt von 1,86 % auf 2,17 % an und weist damit auf die Aktivität der Phospholipase hin. Die Ergebnisse belegen, dass es erstaunlicherweise durch Zusatz eines einzigen weiteren Enzyms, einem Pepsin, zu einer stärkeren Reduktion der Schleimphase kommt und damit die Ölausbeute der Reaktion erhöht wird.

## Patentansprüche

1. Zusammensetzung umfassend eine erste Enzymkomponente umfassend mindestens ein Phospholipid-spaltendes Enzym sowie eine zweite Enzymkomponente umfassend mindestens eine Protease.

2. Zusammensetzung gemäß Anspruch 1, wobei die erste Enzymkomponente ausgewählt wird aus der Gruppe bestehend aus Phospholipase A1, Phospholipase A2, Phospholipase C, Phospholipase B, Phospholipase D, Acyltransferase und deren Mischungen.

3. Zusammensetzung gemäß einem der vorhergehenden Ansprüche, wobei die zweite Enzymkomponente ausgewählt wird aus der Gruppe bestehend aus Aminopeptidasen, Dipeptidasen, Dipeptidylpeptidasen, Tripeptidylpeptidasen, Peptidyldipeptidasen, Carboxypeptidase des Typs Serin, Metallocarboxypeptidasen, Carboxypeptidasen des Typs Cystein, Omegapeptidasen, Serin-Endopeptidasen, Cystein-Endopeptidasen, Asparagin-Endopeptidasen, Metalloendopeptidasen, Threonin-Endopeptidasen, Endopeptidasen und deren Mischungen.

4. Zusammensetzung gemäß einem der vorhergehenden Ansprüche, wobei das Verhältnis der ersten Enzymkomponente zu der zweiten Enzymkomponente im Bereich von 0,001 : 20 bis 20 : 0,001 liegt.

5. Verfahren zur Entschleimung von Triglycerid-haltigen Zusammensetzungen, welches folgende Schritte umfasst:
a) in-Kontakt-Bringen der Triglycerid-haltigen Zusammensetzung mit einer Zusammensetzung wie in einem der Ansprüche 1 bis 4 definiert.
b) Abtrennen der Schleimstoffe von der Triglycerid-haltigen Zusammensetzung.

6. Verfahren gemäß Anspruch 5, wobei die Triglycerid-haltige Zusammensetzung ausgewählt wird aus rohem Pflanzenöl und Pflanzenölschleim.

7. Verfahren gemäß Anspruch 5 oder 6, wobei vor dem in-Kontakt-Bringen gemäß Schritt a) die Triglycerid-haltige Zusammensetzung mit Wasser und/oder wässriger Säure in Kontakt gebracht wird, jedoch vor Schritt a) keine Abtrennung der wässrigen Phase stattfindet.

8. Verfahren gemäß einem der Ansprüche 5 bis 7, wobei die erste und/oder zweite Enzymkomponente in geträgerter Form eingesetzt wird.

9. Verfahren gemäß einem der Ansprüche 5 bis 8, wobei das in-Kontakt-Bringen gemäß Schritt a) des erfindungsgemäßen Verfahrens bei einer Temperatur von 22 bis 70°C erfolgt.

10. Verfahren gemäß einem der Ansprüche 5 bis 9, weiter umfassend den Schritt
c) erneutes in-Kontakt-Bringen der Triglycerid-haltigen Zusammensetzung gemäß Schritt b) mit der ersten und/oder zweiten Enzymkomponente.

11. Verwendung einer Zusammensetzung wie in einem der Ansprüche 1 bis 4 definiert zur Entschleimung Triglyceridhaltiger Zusammensetzungen.
